# EUROPEAN PATENT APPLICATION

(11) **EP 0 823 419 A2**
(43) Date of publication of application: **11.02.1998**
(21) Application number: 97306018.9
(22) Date of filing: 07.08.1997
(51) Int. Cl.: C07C 271/20, C07C 271/06, A61K 7/06

(54) **Dimethylpropanediol compounds**

(30) Priority: 09.08.1996 JP 210875/96
(71) Applicant: TAKEDA CHEMICAL INDUSTRIES, LTD., Chuo-ku, Osaka 541 (JP)
(72) Inventor: Kobayashi, Koji, Kasugai, Aichi 487 (JP); Fukunishi, Hirotada, Yokohama, Kanagawa 222 (JP); Watanabe, Masazumi, Kawanishi, Hyogo 666-01 (JP)
(74) Representative: Lewin, John Harvey

(57) **Abstract**

Disclosed is a novel dimethylpropanediol compound of the formula: 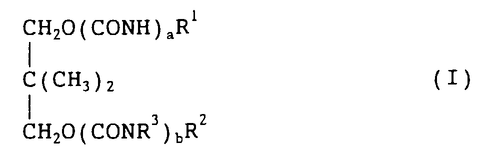 wherein R¹ and R² are each independently a C₁₋₃₀ hydrocarbon group which may be substituted, or a five-or six-membered heterocyclic group which contains 1 to 4 heteroatoms selected from oxygen, sulfur and nitrogen atoms and which may be substituted,
R³ is hydrogen atom, an alkyl group which may be substituted, an acyl group, an alkoxycarbonyl group, a phenoxycarbonyl group, or a carbamoyl group which may be substituted, and
a and b are each independently 0 or 1 or a salt thereof, which has a hair revitalizing action and is useful for hair growth promotion, hair growth stimulation, or hair loss prevention in mammals.

## Description

### BACKGROUND OF THE INVENTION

This invention relates to novel dimethylpropanediol compounds possessing a hair revitalizing action, especially a hair growth promotion or stimulation effect.

Conventionally, hair revitalizing tonic compositions containing various components such as certain vitamins, amino acids, female hormones, and plant extracts having a vasodilative effect or an anti-inflammatory effect have been used for the prophylaxis and treatment of alopecia or prevention or treatment of hair loss. Moreover, a variety of other compounds have also been proposed for use as active ingredients, but they are not always satisfactory from the viewpoint of efficacy. Meanwhile, a large number of 2-substituted glycerol derivatives having antiallergic and anti-inflammatory effects have been synthesized (see, for example, Published Japanese Translation of PCT International Publication No. 501612/'91), and much attention is focused on their excellent effects.

However, there is a still continuing need for a more excellent hair revitalizing tonic composition. Accordingly, it is an object of the present invention to provide a novel compound having a hair revitalizing action, among others, a chemically synthesized compound having a hair growth promotion or stimulation effect.

### SUMMARY OF THE INVENTION

The present inventors have synthesized various compounds and examined their hair growth promotion effects. As a result, it has now been found that novel dimethylpropanediol compounds having two methyl groups at the 2-position have an excellent hair growth promotion effect and that they are low in toxicity.

That is, the present invention relates to
(1) a novel 2,2-dimethylpropanediol compound of the following formula (I) having a hair revitalizing action, especially a hair growth promotion effect: 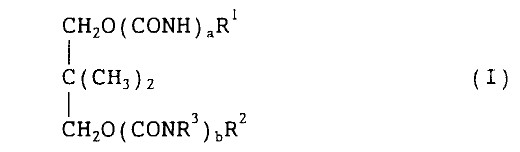 wherein R¹ and R² are each independently a C₁₋₃₀ hydrocarbon group which may be substituted, or a five-or six-membered heterocyclic group which contains 1 to 4 heteroatoms selected from oxygen, sulfur and nitrogen atoms and which may be substituted, R³ is hydrogen, an alkyl group which may be substituted, an acyl group, an alkoxycarbonyl group, a phenoxycarbonyl group, or a carbamoyl group which may be substituted, and a and b are each independently 0 or 1, or a salt thereof,
(2) a compound as described (1), wherein R¹ and R² are each independently a C₁₋₃₀ alkyl group, a C₃₋₈ cycloalkyl group, a C₂₋₁₀ alkenyl group, a C₂₋₁₀ alkynyl group, a C₃₋₁₀ cycloalkenyl group, a C₆₋₁₄ aryl group or a C₇₋₁₆ aralkyl group; when these groups are substituted, 1 to 5 substituents may be present at a substitutable position or positions, and these substituents may be selected from the group consisting of halogen atoms; C₃₋₈ cycloalkyl, hydroxy, mercapto, oxo, thioxo, cyano, carbamoyl, carboxyl, C₁₋₄ alkoxycarbonyl, sulfonyl, C₁₋₄ alkoxy, phenoxy, halogenophenoxy, C₁₋₄ alkylthio, phenylthio, C₁₋₄ alkylsulfinyl, C₁₋₄ alkylsulfonyl, amino, C₁₋₆ acylamino, C₁₋₃₀ alkylamino, di-C₁₋₃₀ alkylamino, C₁₋₅ acyl, benzoyl and C₁₋₁₀ haloalkyl groups; and five- or six-membered heterocyclic groups which contain 1 to 4 heteroatoms selected from oxygen, sulfur and nitrogen atoms in addition to carbon atoms and which may be optionally substituted by 1 to 4 substituents selected from halogen atoms, C₁₋₄ alkyl groups and halogenophenoxy groups, and when R¹ and R² are each a C₃₋₈ cycloalkyl group, a C₃₋₁₀ cycloalkenyl group, a C₆₋₁₄ aryl group or a C₇₋₁₆ aralkyl group, 1 to 4 C₁₋₄ alkyl groups may be present at a position or positions in the ring,
(3) a compound as described (1), wherein R¹ is a C₁₋₃₀ hydrocarbon group,
(4) a compound as described (1), wherein R² is a C₁₋₃₀ hydrocarbon group substituted by an amino group which may be substituted,
(5) a compound as described (1), wherein R² is a group of the formula 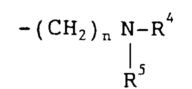 wherein R⁴ and R⁵ are each independently hydrogen atom or a C₁₋₅ alkyl group which may be substituted, and n is an integer of 1 to 10,
(6) a compound as described (5), wherein R⁴ and R⁵ are each independently a C₁₋₃ alkyl group,
(7) a compound as described (1), which is 2,2-dimethyl-3-[(3'-dimethylaminopropyl)carbamoyloxy]-1-(octadecylcarbamoyloxy)propane or a salt thereof, and
(8) a method for producing a compound of the formula (I) as described in (1) wherein b is 1, which comprises reacting a compound of the formula 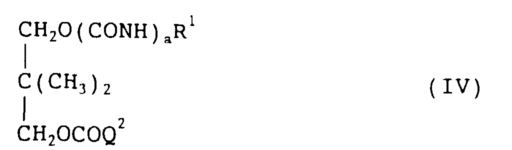 wherein Q² is a group or an atom capable of activating the carbonyl group and the other symbols are as defined in (1), with a compound of the formula

   HN(R³)-R² (V)

   wherein R² and R³ are as defined in (1).

The compounds represented by the above formula (I) and salts thereof exhibit a significantly excellent hair growth promotion effect and, moreover, a hair loss prevention effect and a dandruff and itchy scalp suppression effect. Accordingly, the term "hair revitalization" or "hair revitalizing action" as used herein means at least one of the above-described effects.

### DETAILED DESCRIPTION OF THE INVENTION

The compounds of the formula (I) are more specifically described hereinbelow.

The C₁₋₃₀ hydrocarbon groups represented by R¹ and R² in formula (I) include, for example, C₁₋₃₀ alkyl groups, C₃₋₈ cycloalkyl groups, C₂₋₁₀ alkenyl groups, C₂₋₁₀ alkynyl groups, C₃₋₁₀ cycloalkenyl groups, C₆₋₁₄ aryl groups and C₇₋₁₆ aralkyl groups.

Examples of the C₁₋₃₀ alkyl groups include straight-chain alkyl groups such as methyl, ethyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl and triacontyl; and branched alkyl groups such as isopropyl, isobutyl, sec-butyl, tert-butyl, 2-methylpentyl, 3-methylpentyl, 4-isocapryl, 4-ethylpentyl, 6-methyldecyl, 9-methyldecyl, 6-ethylnonyl, 5-propyloctyl, 11-methyldodecyl, 12-methyldodecyl, 4-methyltetradecyl, 13-methyltetradecyl, 14-ethylhexadecyl, 10-methyloctadecyl, 15-ethylpentadecyl, 10-methyldocosyl, 2-pentyloctadecyl, 22-methyltricosyl, 12-hexyloctadecyl, 6-methyltetracosyl, 24-methylheptacosyl, 2-decylhexadecyl, 2-nonyloctadecyl, 2-dodecyloctadecyl, 3-methyltetracosyl and 3-methyltricosyl.

Preferred examples of the groups represented by R¹ and R² are straight-chain or branched C₆₋₂₂ alkyl groups which may be substituted, and more preferred examples thereof are straight-chain or branched C₈₋₂₀ alkyl groups.

Examples of the C₃₋₈ cycloalkyl groups include cyclopropyl, cyclopentyl and cyclohexyl. Examples of the C₂₋₁₀ alkenyl groups include vinyl, allyl, 2-methylallyl, 2-butenyl, 3-butenyl, 3-octenyl, 2-nonenyl and 4-decenyl. Examples of the C₂₋₁₀ alkynyl groups include ethynyl, 2-propynyl and 3-hexynyl.

Examples of the C₃₋₁₀ cycloalkenyl groups include cyclopropenyl, cyclopentenyl and cyclohexenyl. Examples of the C₆₋₁₄ aryl groups include phenyl and naphthyl. Examples of the C₇₋₁₆ aralkyl groups include benzyl, phenylethyl and naphthylmethyl.

The foregoing groups represented by R¹ and R² may have one or more (e.g., one to five) substituents at any substitutable position or positions in the carbon chain or carbon ring. Specific examples of the substituents include halogen atoms (e.g., fluorine, chlorine, bromine and iodine) and C₃₋₈ cycloalkyl, hydroxy, mercapto, oxo, thioxo, cyano, carbamoyl, carboxyl, C₁₋₄ alkoxycarbonyl (e.g., methoxycarbonyl and ethoxycarbonyl), sulfonyl, C₁₋₄ alkoxy (e.g., methoxy, ethoxy, propoxy, isopropoxy, butoxy, isobutoxy, sec-butoxy and tert-butoxy), phenoxy, halogenophenoxy (e.g., o-, m- or p-chlorophenoxy and o-, m- or p-bromophenoxy), lower (C₁₋₄) alkylthio (e.g., methylthio, ethylthio, n-propylthio, isopropylthio, n-butylthio and tert-butylthio), phenylthio, C₁₋₄ alkylsulfinyl (e.g., methylsulfinyl and ethylsulfinyl), C₁₋₄ alkylsulfonyl (e.g., methylsulfonyl and ethylsulfonyl) and C₁₋₁₀ haloalkyl groups (e.g., difluoromethyl, trifluoromethyl, trifluoroethyl and trichloroethyl). Other specific examples of the substituents for the hydrocarbon groups include amino; substituted amino such as C₁₋₆ acylamino (e.g., acetylamino and propionylamino), C₁₋₃₀ alkylamino (e.g., methylamino, ethylamino, n-propylamino, isopropylamino, n-butylamino, isobutylamino, s-butylamino, t-butylamino, pentylamino, hexylamino, heptylamino, octylamino, nonylamino, decylamino, undecylamino, dodecylamino, tridecylamino, tetradecylamino, pentadecylamino, hexadecylamino, heptadecylamino, octadecylamino, nonadecylamino, icosylamino, henicosylamino, docosylamino, tricosylamino, tetracosylamino, pentacosylamino, hexacosylamino, heptacosylamino, octacosylamino, nonacosylamino and triacontylamino), di-C₁₋₄ alkylamino (e.g., dimethylamino, diethylamino, N-methyl-N-ethylamino and N-methyl-N-propylamino); C₁₋₅ acyl (e.g., C₁₋₅ alkanoyl such as formyl, acetyl and propionyl); and benzoyl groups.

Further specific examples of the substituents for the hydrocarbon groups include five- and six-membered heterocyclic groups which contain 1 to 4 heteroatoms selected from oxygen, sulfur and nitrogen atoms and which may be substituted, such as pyrrolidyl, piperidyl, morpholino, thiomorpholino, 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 3-, 4- or 5-isoxazolyl, 2-, 4- or 5-imidazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl, imidazoquinolyl and indolyl. These heterocyclic groups are usually linked to C₁₋₂ hydrocarbon groups. These heterocyclic groups may have 1 to 4 substituents. Specific examples of these substituents include halogen atoms (e.g., fluorine, chlorine and bromine), C₁₋₄ alkyl groups (e.g., methyl, ethyl, propyl and isopropyl) and halogenophenoxy groups (e.g., o-, m- or p-chlorophenoxy and o-, m- or p-bromophenoxy).

Preferred substituents for the aforesaid cycloalkyl, cycloalkenyl, aryl and aralkyl groups include C₁₋₄ alkyl groups such as methyl, ethyl, propyl, isopropyl and butyl, and they may have one to four substituent selected from such alkyl groups. Where the aforesaid alkyl groups are substituted by a C₃₋₈ cycloalkyl group or groups, it is preferable that the alkyl groups are straight-chain C₆₋₁₄ alkyl groups.

Examples of the heterocyclic groups represented by R¹ and R² include the same five- and six-membered heterocyclic groups which contain 1 to 4 heteroatoms selected from oxygen, sulfur and nitrogen atoms as those mentioned above as the substituents for the hydrocarbon groups represented by R¹ and R². Preferred examples of the heterocyclic groups represented by R¹ and R² include five- or six-membered aromatic heterocyclic groups and alicyclic heterocyclic groups, such as 2-, 3- or 4-pyridyl and 2-, 3- or 4-piperidyl. These heterocyclic groups may be substituted, preferably, by 1 to 4 substituents selected from halogen atoms and C₁₋₄ alkyl groups.

Especially preferred compounds are those in which, among the foregoing R¹ and R² groups, R¹ is a C₁₋₃₀ hydrocarbon group and R² is a C₁₋₃₀ hydrocarbon group substituted by an amino group that may be substituted, or salts thereof. In particular, the compounds in which R² is a group of the formula 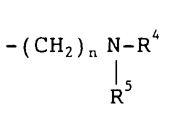 wherein R⁴ and R⁵ are each independently a hydrogen atom or a C₁₋₅ alkyl group which may be substituted, and n is an integer of 1 to 10, or salts thereof are preferred. It is preferable that R⁴ and R⁵ represent C₁₋₃ alkyl groups which may be the same or different.

Useful substituents for the C₁₋₅ alkyl group include, for example, hydroxy groups. The C₁₋₅ alkyl group may have 1 to 3 substituents.

In the above formula, R⁴ and R⁵ may form a nitrogen-containing heterocyclic ring in conjunction with the nitrogen atom to which they are linked. Specifically, R⁴ and R⁵ may be combined to form a tetramethylene or pentamethylene chain. Alternatively, R⁴ and R⁵ may form a five- or six-membered heterocyclic ring through conecting with the adjacent nitrogen atom and one or more other heteroatoms (e.g., oxygen, nitrogen and/or sulfur atoms).

In the above formula, examples of the alkyl group represented by R³ include C₁₋₅ alkyl groups such as methyl, ethyl, propyl, butyl and pentyl. These alkyl groups may be substituted, for example, by 1 to 4 substituents selected from carboxyl and lower (C₁₋₅) alkoxycarbonyl (e.g., methoxycarbonyl, ethoxycarbonyl, propoxycarbonyl, butoxycarbonyl or pentoxycarbonyl) groups. Examples of the acyl group represented by R³ include lower (C₁₋₅) alkanoyl (e.g., formyl, acetyl, propionyl, butyryl, isobutyryl, valeryl and isovaleryl) and benzoyl groups. Examples of the alkoxycarbonyl group represented by R³ include methoxycarbonyl, ethoxycarbonyl, propoxycarbonyl, butoxycarbonyl and pentoxycarbonyl. Examples of the carbamoyl group which may be substituted, as represented by R³, include carbamoyl, lower (C₁₋₅) alkylcarbamoyl (e.g., methylcarbamoyl, ethylcarbamoyl, propylcarbamoyl and butylcarbamoyl), di-lower (C₁₋₅) alkylcarbamoyl (e.g., dimethylcarbamoyl, methylethylcarbamoyl, diethylcarbamoyl and methylpropylcarbamoyl), and three-to seven-membered cyclic aminocarbonyl [e.g., (aziridin-1-yl)carbonyl, (azetidin-1-yl)carbonyl, (pyrrolidin-1-yl)carbonyl, piperidinocarbonyl, (piperazin-1-yl)carbonyl, morpholinocarbonyl and thiomorpholinocarbonyl].

The alkyl group represented by R³ may be combined with R⁴ or R⁵ to form an alkenylene or alkylene bridge. Specific examples of the alkenylene or alkylene bridge include lower (C₁₋₄) alkenylene and alkylene groups such as methylene, ethylene, trimethylene, tetramethylene, vinylene and propenylene. These groups may have one or two substituents (e.g., oxo) at a substitutable position or positions. Examples of substituted alkylene and alkenylene groups include 1-oxoethylene, 3-oxopropenylene and 1,2-dioxoethylene. In this case, specific examples of the groups which R³ forms in conjunction with R⁴ or R⁵ together with the adjacent nitrogen atom include groups of the formulae

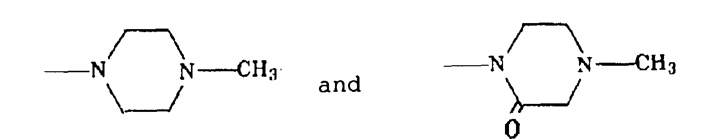

Preferably, R³ is hydrogen or lower alkanoyl. More preferably, R³ is hydrogen.

When both of a and b are 0, the compounds of the formula (I) are such that R¹ and R² are each joined through an ether linkage. When both of a and b are 1, R¹ and R² are joined through an oxycarbamoyl linkage (-OCONH-) and -OCONR³-, respectively. The compounds in which one of a and b is 0 and the other is 1 also fall within the scope of the formula (I).

When the compounds of the formula (I) have asymmetric carbon atoms in R¹ or R², the compounds may be in optically active forms or in a mixture of optically active forms. Also, the compounds of the formula (I) may be hydrates or not be hydrates.

Where the compounds of the formula (I) have a basic group such as an amino or imino group, the compounds of the formula (I) can be acid addition salts thereof. Preferred examples of such salts include pharmaceutically or cosmetically acceptable inorganic acid salts such as hydrohalogenides (e.g., hydrochloride and hydrobromide), sulfate, nitrate and phosphate; and pharmaceutically or cosmetically acceptable organic acid salts such as acetate, propionate, hydroxyacetate, 2-hydroxypropionate, 2-oxopropionate, ethanedicarboxylate, propanedicarboxylate, butanedicarboxylate, methanesulfonate, ethanesulfonate, benzenesulfonate, 4-methylbenzenesulfonate and 2-hydroxybenzoate. Where the compounds of the formula (I) have a carboxyl group, it can also be used as ammonium salts, alkali metal salts (e.g., lithium, sodium and potassium salts), or salts with organic bases (e.g., amino acids such as arginine and lysine).

The compounds of the formula (I) or salts thereof (hereinafter sometimes referred to as compounds (I) including compounds (I) and their salts) may be prepared, for example, in the following manner.
A) Compounds (I) can be obtained by reacting a compound (II) of the formula 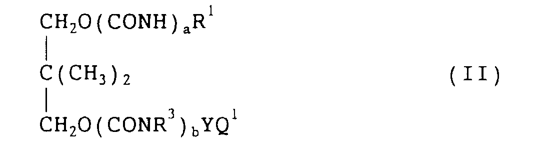 wherein Y is a lower alkylene group which may be substituted, Q¹ is a group capable of being easily replaced by nitrogen [such as halogen (e.g., chlorine, bromine or iodine), O-tosyl or O-mesyl], and the other symbols are as defined previously, with an amine (III) of the formula

   NHR⁴R⁵ (III)

   wherein the symbols are as defined previously, which may be substituted. This reaction may be carried out by adding an equivalent amount or a large excess of compound (III) to compound (II), and reacting this mixture in the presence or absence of a solvent at a temperature of 0 to 200°C for about 0.5-48 hours. Usable solvents include toluene, benzene, ether, dioxane, tetrahydrofuran and the like, and compound (III) itself may be used as the solvent. Under heated conditions, the reaction may be carried out in a sealed tube.
B) Compounds (I) can be obtained by reacting a compound (IV) of the formula 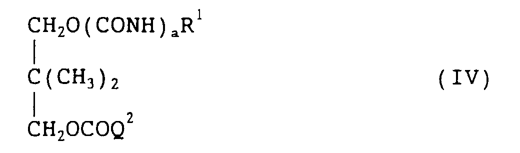 wherein Q² is a group or an atom (hereinafter referred to briefly as a group) capable of activating the carbonyl group [such as halogen (e.g., chlorine) or phenoxy] and the other symbols are as defined previously, with a compound (V) of the formula

   HN(R³)-R² (V)

   wherein R² is as defined above including -W-Z (wherein W is a single bond or a lower (C₁₋₁₀) alkylene group, and Z is a mono- or di-substituted amino group or, when W is a single bond, a five- or six-membered nitrogen-containing heterocyclic group attached to a carbon atom), and the other symbol is as defined previously. The mono- or di-substituted amino groups represented by Z include the same ones as described as the substituents for the hydrocarbon groups represented by R¹ and R². The reaction of compound (IV) with compound (V) may be carried out in the presence or absence of a solvent at a temperature of -10 to 150°C for about 0.5-48 hours. Usable solvents include toluene, benzene, ether, dioxane, tetrahydrofuran, chloroform and the like. In order to accelerate the reaction, a base such as triethylamine or pyridine may be added. It is also possible to react compound (V) with sodium hydride, n-butyllithium or the like in any of the above-described solvents to convert it into a metallic salt thereof, and then react this metallic salt with compound (IV).
C) Compounds (I) can be obtained by reacting a compound (VI) of the formula 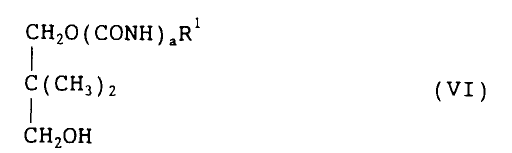 wherein the symbols are as defined previously, with a compound (VII) of the formula

   Q²-CO-N(R³)-R² (VII)

   wherein the symbols are as defined previously. In the formula (VII), R² includes the group-W-Z (wherein the symbols are as defined above).

The reaction of compound (VI) with compound (VII) may be carried out in substantially the same manner as described for the reaction of compound (IV) with compound (V) in B).
D) Compounds of the formula (I) in which b is 0 and R² is -(CH₂)ₙNR⁴R⁵ can be obtained by reacting a compound (VIII) of the formula 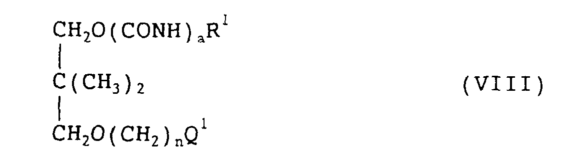 wherein the symbols are as defined previously, with a compound (III) of the formula

   NHR⁴R⁵ (III)

   wherein R⁴ and R⁵ are as defined previously. This reaction may be carried out in substantially the same manner as described for the reaction of compound (II) with compound (III) in A).
E) Compounds (I) can be obtained by reacting a compound of the formula

   R²NCO (IX)

   wherein the symbol is as defined previously, with a compound of formula (VI). This reaction may be carried out in the presence or absence of a solvent at a temperature of -10 to +150°C for about 0.5-48 hours.
Usable solvents include toluene, benzene, ether, dioxane, tetrahydrofuran, chloroform and the like. In order to accelerate the reaction, a base such as triethylamine, pyridine or 4-dimethylaminopyridine may be added.

Compound (IX) may readily be synthesized, for example, by reacting a compound (X) of the formula

R²NH₂ (X)

wherein the symbol is as defined previously, with diphosgene in the absence of solvent or in an inert solvent such as methylene chloride, chloroform, benzene or tetrahydrofuran, at a temperature of -20 to 120°C for about 0.5-12 hours, or by reacting a compound (XI) of the formula

R²COOH (XI)

wherein the symbol is as defined previously, with diphenyl phosphorylazide (DPPA) in a solvent such as chloroform, toluene, benzene, dichloromethane or tetrahydrofuran, in the presence of a tertiary amine such as triethylamine or tributylamine, at a temperature of 0 to 150°C for about 0.5-48 hours, and then in the presence of a tertiary amine such as pyridine, at a temperature of 0 to 150°C for about 0.5-48 hours.

A compound of the formula (I) in which the nitrogen atom contained in the R² substituent group is secondary or tertiary can be obtained by reacting a compound of the formula (I) in which the nitrogen atom contained in the R² substituent group is primary or secondary, for example, with an alkyl halide. This reaction may be carried out in a solvent such as ether, chloroform, tetrahydrofuran, benzene or toluene, in the presence of an equivalent amount or large excess of an alkyl halide, at a temperature of 0 to 150°C for about 0.5-60 hours.

The synthesis intermediate (VIII) can be obtained, for example, by reacting a compound (VI) of the formula 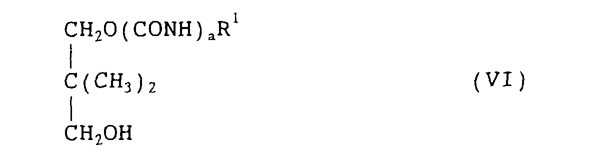 wherein the symbols are as defined previously, with a dihaloalkane of the formula

Hal(CH₂)ₙHal (XII)

wherein Hal represents a halogen atom such as chlorine and bromine and the other symbol is as defined above. This reaction may be carried out in the absence of solvent or in a suitable solvent (e.g., benzene, toluene, hexane, dioxane or tetrahydrofuran), in the presence of a strong base (e.g., sodium hydroxide, potassium hydroxide or an aqueous solution thereof) and preferably in the presence of a phase-transfer catalyst (e.g., cetyltrimethylammonium chloride or benzyltrimethylammonium chloride) when water is contained in the reaction system, at a temperature of -20 to 150°C and preferably 20 to +100°C for about 0.5-60 hours.

The starting compound (VI) may be prepared according to the following reaction formula. 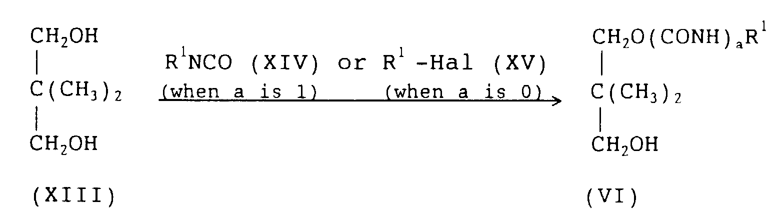 wherein the symbols are as defined previously.

The reaction of compound (XIII) with alkyl isocyanate (XIV) may be carried out in the presence or absence of a solvent at a temperature of -10 to +150°C for about 0.5-48 hours. Usable solvents include toluene, benzene, ether, dioxane, tetrahydrofuran, chloroform and the like. In order to accelerate the reaction, a base such as triethylamine, pyridine or 4-dimethylaminopyridine may be added. The reaction of compound (XIII) with alkyl halide (XV) may be carried out in substantially the same manner as described for the reaction of compound (VI) with compound (XII).

In this reaction, a compound (XVI) of the formula 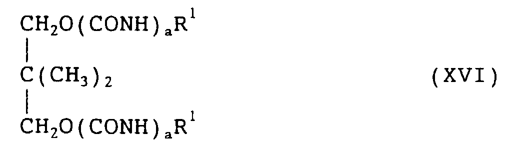 wherein the symbols are as defined previously, [i.e., a compound of the formula (I) in which R¹ and R² are the same, a and b are the same, and R³ is hydrogen atom] can be obtained by reacting one mole of the starting compound (XIII) with two or more moles of compound (XIV) or (XV).

The starting compound (IV) can be produced by reacting compound (VI) with phenyl halogenocarbonate (e.g., phenyl chlorocarbonate) in accordance with the similar reaction conditions as described in method E).

In each of the above-mentioned reactions, when the starting compounds and intermediate compounds has amino group, carboxyl group or hydroxy group as the substituent, they may have a protective group generally used in the peptide chemistry. After completion of the reaction, the target compound can be obtained by removing the protective group upon necessity.

Examples of the amino-protecting group include optionally substituted C₁₋₆ alkyl carbonyl (e.g. formyl, methyl carbonyl and ethyl carbonyl), phenyl carbonyl, C₁₋₆ alkyl-oxycarbonyl (e.g. methoxycarbonyl and ethoxycarbonyl), phenyloxycarbonyl (e.g. benzoxycarbonyl), C₇₋₁₀ aralkyloxycarbonyl (e.g. benzyloxycarbonyl), trityl and phthaloyl. Examples of substituents of them include halogen atoms (e.g. fluoro, chloro, bromo and iodo), C₁₋₆ alkyl-carbonyl (e.g. methylcarbonyl, ethylcarbonyl and butylcarbonyl) and nitro group, and the number of the substituents ranges from about 1 to 3.

Examples of the carboxyl-protecting group include C₁₋₆ alkyl (e.g. methyl, ethyl, n-propyl, i-propyl, n-butyl and tert-butyl), phenyl, trityl and silyl. Examples of substituents of them include halogen atoms (e.g. fluorine, chlorine, bromine and iodine), C₁₋₆ alkylcarbonyl (formyl, methylcarbonyl, ethylcarbonyl and butylcarbonyl) and nitro group, and the number of the substituents ranges from about 1 to 3.

Examples of the hydroxy-protecting group include for example, optionally substituted C₁₋₆ alkyl (e.g. methyl, ethyl, n-propyl, i-propyl, n-butyl and tert-butyl), phenyl, C₇₋₁₀ aralkyl (e.g. benzyl), C₁₋₆ alkylcarbonyl (e.g. formyl, methylcarbonyl and ethylcarbonyl), phenyloxycarbonyl, C₇₋₁₀ aralkyloxycarbonyl (e.g. benzyloxycarbonyl), pyranyl, furanyl and silyl. As the substituents mentioned above, halogen atoms (e.g. fluoro, chloro, bromo and iodo), C₁₋₆ alkyl, phenyl, C₇₋₁₀ aralkyl and nitro group were used. The number of substituents ranges from about 1 to 4.

And, the protecting groups can be introduced and removed by per se known means or those analogous thereto (for example, I.F.W. McOmie et al., PROTECTIVE GROUPS IN ORGANIC CHEMISTRY, Plenum Press). More specifically, those protecting groups are removed by, for example, acid, base, reduction, ultraviolet ray, hydrazine, phenylhydrazine, sodium N-methyldithiocarbamate, tetrabutyl ammonium fluoride or palladium acetate.

The compound (I) produced by the above-mentioned methods can be isolated and purified by a conventional separating means such as recrystallization, distillation and chromatography. When the compound (I) thus obtained is in the free form, it can be converted to a salt by per se known means or analogous means thereto (e.g. neutralization). Conversely, when the compound (I) is obtained in the form of a salt, it can be converted to the free form or any other salt by per se known means or analogous means thereto.

Although several typical processes for the preparation of compounds (I) have been described above, compounds (I) may also be prepared according to modifications of these processes.

The compounds (I) have very low toxicity and therefore can be safely applied to mammals including humans, monkeys, dogs, cats and horses. The compounds (I) exhibit hair revitalizing actions, especially hair growth promotion or stimulation effect and therefore are useful as active components for hair revitalization, especially as hair revitalizing tonic compositions. Such hair revitalizing tonic composition may comprise not only compounds (I) but also pharmaceutically or cosmetically acceptable vehicles, carriers and adjuvants or other active components in amounts within a range which does not impair the effects of the present invention. Such adjuvants include adjuvants which are considered to have a hair generating effect and the like in themselves. Examples of these additional components include plant extracts such as swertia herb extract and ginseng extract; vitamins such as vitamin B6, vitamin E and derivatives thereof, and biotin; hair generating agents and hair generating aids such as pantothenic acid and derivatives thereof, glycyrrhizinic acid and derivatives (e.g. monoammonium glycyrrhizinate) thereof, glycyrrhetinic acid and derivatives thereof, nicotinic acid esters (e.g., benzyl nicotinate), cyclosporins, carpronium chloride, cepharanthine, oxendolone, diazoxide, minoxidil and ethynylestradiol; antibacterial agents such as hinokitiol, hexachlorophene, phenol, isopropylmethylphenol, benzalkonium chloride, cetylpyridinium chloride, undecylenic acid, trichlorocarbanilide and bithionol; refrigerants such as menthol and eucalyptus oil; drugs such as salicylic acid, zinc and derivatives thereof, and lactic acid and alkyl esters thereof; organic acids such as citric acid, succinic acid and malic acid; amino acids such as arginine; oil components such as silicone oil, olive oil, squalane, vaseline, liquid paraffin, higher fatty acid esters (e.g. isopropyl myristate, isocetyl octanoate), higher fatty acids (e.g. stearic acid) and higher alcohols (e.g. cetanol, cetostearyl alcohol); polyhydric alcohols such as glycerin, propylene glycol 1,3-butylene glycol, dipropylene glycol and polyethylene glycol; esters of polyhydric alcohols such as glyceryl monostearate; surfactants such as alkylsulfates, hydrogenated castor oil ethylene oxide adduct (polyoxyethylene hydrogenated castor oil), cocoacyl N-methyltaurate, polyoxyethylene alkyl ether, fatty acid diethanolamides, ethylene glycol fatty acid esters, stearyltrimethylammonium salts, alkyltrimethylammonium chloride sorbitan monooleate and amine oxide; high molecular compounds such as carboxymethylcellulose; sequestering agents such as hexametaphosphate and edetic acid and salts thereof (e.g. disodium edetate); perfumes; antioxidants; ultraviolet absorbers; dyes; ethanol; water; humectants; thickeners; and preservatives.

Especially, the above-mentioned antibacterial agents are preferably used in combination with compounds (I).

The hair revitalizing tonic compositions containing the compound (I) of the present invention may be prepared in any dosage form that can be applied to the integument, such as a liquid, emulsion, ointment, cream, gel or aerosol, and may be provided in various product forms such as tonics, conditioners and scalp treatments. These hair revitalizing tonic compositions can be prepared by mixing various ingredients, followed by solubilizing or kneading of the mixture, using any of the apparatus and methods commonly used in the relevant technical field.

The compounds (I) may be administered in such dosage forms in a concentration of 0.001-95 w/w %, preferably 0.01-30 w/w %.

Since compounds (I) have very low toxicity to human beings as mentioned above, hair revitalizing tonic compositions containing compounds (I) of the present invention can be percutaneously administered by applying them directly to the skin or hair or spraying them directly over the skin or hair. The dosage of these hair revitalizing tonic compositions cannot be definitely determined because it may vary with the age, individual, severity of symptoms, and the like. For human beings, however, compounds (I) are generally administered in a daily dose of 0.01 to 100 mg, preferably 0.1 to 50 mg, per kg of body weight. This dose may be administered once a day or in two to four divided doses.

The action and effects of compounds (I) of the present invention can be confirmed by evaluating the hair generating effect of each compound and putting it to practical use on the basis of the results thus obtained, if necessary.

Thus, the present invention provides the new compounds (I) having hair revitalizing action for mammals (including man) requiring a hair revitalization or hair loss prevention treatment and therefore useful for hair revitalizing tonic compositions. More specifically, compounds (I) of the present invention are highly effective for the promotion of hair growth, the stimulation of hair growth, the prevention of hair loss, the prevention of an itchy scalp, and the prevention of dandruff.

The present invention is more specifically explained with reference to the following reference example, working examples and formula example.

### Reference Example 1

### 2,2-Dimethyl-3-(octadecylcarbamoyloxy)-1-propanol

To a stirred methylene chloride solution (15 ml) containing 2.08 g (20.00 mmol) of 2,2-dimethyl-1,3-propanediol and 2.12 g (21.00 mmol) of triethylamine was added dropwise 5.91 g (20.00 mmol) of octadecyl isocyanate at room temperature, followed by stirring at room temperature for 21 hours. The reaction mixture was diluted with chloroform (15 ml) and filtered to remove any insoluble matter. The filtrate was washed with 1N hydrochloric acid, a saturated aqueous solution of sodium hydrogen carbonate, and a saturated aqueous solution of sodium chloride. After being dried over anhydrous magnesium sulfate, the filtrate was concentrated under reduced pressure. The resulting residue was separated and purified by silica gel (250 ml) column chromatography (chloroform : methanol = 50 : 1 to 20 : 1) to obtain 6.56 g of the title compound (in a 82% yield).

### Example 1

### 2,2-Dimethyl-3-[(3'-dimethylaminopropyl)carbamoyloxy]-1-(octadecylcarbamoyloxy)propane

To a stirred methylene chloride solution (35 ml) containing 6.12 g (15.31 mmol) of 2,2-dimethyl-3-octadecylcarbamoyloxy-1-propanol and 2.42 g (30.62 mmol) of pyridine was added dropwise 2.40 g (15.31 mmol) of phenyl chlorocarbonate under cooling with ice, followed by stirring at room temperature for 1.5 hours. The reaction mixture was washed with a saturated aqueous solution of sodium hydrogen carbonate, dried over anhydrous magnesium sulfate, and then concentrated under reduced pressure. To the resulting residue was added 1.56 g (15.31 mmol) of N,N-dimethyl-1,3-propanediamine, followed by stirring at 70°C for 5 hours. The reaction mixture was separated and purified by silica gel (200 ml) column chromatography (chloroform : methanol = 100 : 1 to 10 : 1) to obtain 7.57 g of the title compound (in a 94% yield). This compound (470 mg) was dissolved in a solvent mixture composed of 0.5 ml of acetone and 3 ml of hexane by the application of heat, and recrystallized by allowing the resulting solution to stand at 0°C for 6 hours. Thus, there was obtained 220 mg of a purified product (with a purity of 98.4% as expressed in terms of an HPLC area percentage).
Melting point: 53.2-53.8°C
¹H-NMR (CDCl₃) δ:
0.88 (3H, t, J=6.8 Hz)
0.93 (6H, s)
1.25 (30H, m)
1.48 (2H, m)
1.66 (2H, m)
2.23 (6H, s)
2.35 (2H, t, J=6.8 Hz)
3.15 (2H, m)
3.24 (2H, dt, J=6.1, 6.3 Hz)
3.86 (4H, m)
4.69 (1H, brs)
5.49 (1H, brs)

### Example 2

### 2,2-Dimethyl-3-[(3'-dimethylaminopropyl)carbamoyloxy]-1-(octadecylcarbamoyloxy)propane hydrochloride

To a stirred ethyl acetate solution (40 ml) containing 5.96 g (11.29 mmol) of 2,2-dimethyl-3-[(3'-dimethylaminopropyl)carbamoyloxy]-1-(octadecylcarbamoyloxy)propane was added dropwise 2.96 ml (11.86 mmol) of a 4N hydrogen chloride solution in ethyl acetate under cooling with ice, followed by stirring for 0.5 hour under cooling with ice. The resulting precipitate was separated by filtration and washed with ethyl acetate to obtain 6.05 g of the title compound (in a 95% yield). Recrystallization of this compound from a solvent mixture composed of ethanol and ethyl acetate was repeated four times to obtain 2.51 g of a purified product (with a purity of 99.0% as expressed in terms of an HPLC area percentage).
Melting point: 83.0-84.0°C
¹H-NMR (CDCl₃) δ:
0.88 (3H, t, J=6.9 Hz)
0.94 (6H, s)
1.25 (30H, m)
1.49 (2H, m)
2.09 (2H, dt, J=6.5, 7.0 Hz)
2.81 (3H, s)
2.82 (3H, s)
3.10-3.17 (4H, m)
3.37 (2H, dt, J=5.9, 6.1 Hz)
3.86 (4H, m)
4.81 (1H, brs)
5.72 (1H, brs)

It was confirmed that the compounds obtained in accordance with the above Examples 1 and 2 have excellent hair growth promotion effects as compared with the control compounds in a hair growth test with the use of mice.

### Formula Example 1

A lotion having the following composition was prepared.

| | (wt.%) |
|---|---|
| 95% ethanol | 50.0 |
| Monoammonium glycyrrhizinate | 0.1 |
| Compound obtained in Example 2 | 1.0 |
| Sodium lauryl sulfate | 0.1 |
| Hydrogenated castor oil ethylene oxide (40 mole) adduct | 0.5 |
| Succinic acid | q.s. |
| Perfume and dye | q.s. |
| Purified water | Balance |

### (Preparation method)

Hydrogenated castor oil ethylene oxide (40 mole) adduct and a perfume were dissolved in 95% ethanol, followed by the addition of purified water. Then, other ingredients were added thereto and dissolved therein with stirring to obtain a clear liquid lotion.

## Claims

1. A compound of the formula 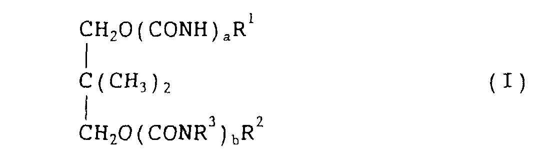 wherein
R¹ and R² are each independently a C₁₋₃₀ hydrocarbon group which may be substituted, or a five-or six-membered heterocyclic group which contains 1 to 4 heteroatoms selected from oxygen, sulfur and nitrogen atoms and which may be substituted,
R³ is hydrogen atom, an alkyl group which may be substituted, an acyl group, an alkoxycarbonyl group, a phenoxycarbonyl group, or a carbamoyl group which may be substituted, and
a and b are each independently 0 or 1, or a salt thereof.

2. A compound as claimed in claim 1 wherein R¹ and R² are each independently a C₁₋₃₀ alkyl group, a C₃₋₈ cycloalkyl group, a C₂₋₁₀ alkenyl group, a C₂₋₁₀ alkynyl group, a C₃₋₁₀ cycloalkenyl group, a C₆₋₁₄ aryl group or a C₇₋₁₆ aralkyl group; when these groups are substituted, 1 to 5 substituents may be present at a substitutable position or positions, and these substituents may be selected from the group consisting of halogen atoms; C₃₋₈ cycloalkyl, hydroxy, mercapto, oxo, thioxo, cyano, carbamoyl, carboxyl, C₁₋₄ alkoxycarbonyl, sulfonyl, C₁₋₄ alkoxy, phenoxy, halogenophenoxy, C₁₋₄ alkylthio, phenylthio, C₁₋₄ alkylsulfinyl, C₁₋₄ alkylsulfonyl, amino, C₁₋₆ acylamino, C₁₋₃₀ alkylamino, di-C₁₋₃₀ alkylamino, C₁₋₅ acyl, benzoyl and C₁₋₁₀ haloalkyl groups; and five- or six-membered heterocyclic groups which contain 1 to 4 heteroatoms selected from oxygen, sulfur and nitrogen atoms in addition to carbon atoms and which may be optionally substituted by 1 to 4 substituents selected from halogen atoms, C₁₋₄ alkyl groups and halogenophenoxy groups, and when R¹ and R² are each a C₃₋₈ cycloalkyl group, a C₃₋₁₀ cycloalkenyl group, a C₆₋₁₄ aryl group or a C₇₋₁₆ aralkyl group, 1 to 4 C₁₋₄ alkyl groups may be present at a position or positions in the ring.

3. A compound as claimed in claim 1 wherein R¹ is a C₁₋₃₀ hydrocarbon group.

4. A compound as claimed in claim 1 wherein R² is a C₁₋₃₀ hydrocarbon group substituted by an amino group which may be substituted.

5. A compound as claimed in claim 1 wherein R² is a group of the formula 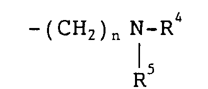 wherein R⁴ and R⁵ are each independently hydrogen atom or a C₁₋₅ alkyl group which may be substituted, and n is an integer of 1 to 10.

6. A compound as claimed in claim 5 wherein R⁴ and R⁵ are each independently a C₁₋₃ alkyl group.

7. A compound as claimed in claim 1 which is 2,2-dimethyl-3-[(3'-dimethylaminopropyl)carbamoyloxy]-1-(octadecylcarbamoyloxy)propane or a salt thereof.

8. A method for producing a compound of the formula (I) as claimed in claim 1 wherein b is 1, which comprises reacting a compound of the formula. 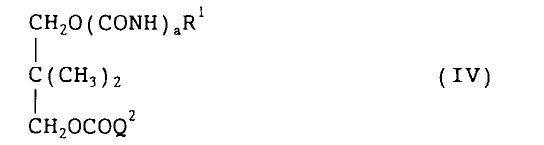 wherein Q² is a group or an atom capable of activating the carbonyl group and the other symbols are as defined in claim 1, with a compound of the formula
HN(R³)-R² (V)
wherein R² and R³ are as defined in claim 1.
